(19) Europäisches Patentamt
European Patent Office
Office européen des brevets

(11) Numéro de publication: 0 140 783
B1

(12) # FASCICULE DE BREVET EUROPÉEN

(45) Date de publication du fascicule du brevet: 26.11.86

(51) Int. Cl.⁴: C 07 C 93/14

(21) Numéro de dépôt: 84402102.2

(22) Date de dépôt: 19.10.84

(54) **Procédé de préparation d'anilines méta substituées.**

(30) Priorité: 28.10.83 FR 8317246

(43) Date de publication de la demande: 08.05.85 Bulletin 85/19

(45) Mention de la délivrance du brevet: 26.11.86 Bulletin 86/48

(84) Etats contractants désignés: AT BE CH DE FR GB IT LI LU NL SE

(56) Documents cité:
FR-A-2 136 287

METHODEN DER ORGANISCHEN CHEMIE, (Houben-Weyl), vol. XI/1, 1957, édition 4, éditeur Eugen Müller, Georg Thieme Verlag, "Stickstoffverbindungen II", pages 77-79, Stuttgart, DE;
METHODEN DER ORGANISCHEN CHEMIE, (Houben-Weyl), vol. VI/3, partie 3, édition 4, éditeur Eugen Müller, 1965, "Sauerstoffverbindungen I, Teil 3", pages 62-66, Georg Thieme Verlag, Stuttgart, DE;
METHODEN DER ORGANISCHEN CHEMIE, (Houben-Weyl), vol. V/3, édition 4, éditeur Eugen Müller, 1962, "Halogenverbindungen", pages 121-123, 606-607, Georg Thieme Verlag, Stuttgart, DE;
J. ORG. CHEM., vol. 44, no. 16, 1979, pages 2907-2910, American Chemical Society, Washington,

(73) Titulaire: RHONE- POULENC SPECIALITES CHIMIQUES, "Les Miroirs" 18, Avenue d'Alsace, F-92400 Courbevoie (FR)

(72) Inventeur: Langlois, Bernard, 91, rue Du Guesclin, F-69006 Lyon (FR)
Inventeur: Soula, Gérard, 33, rue Nungesser, F-69330 Meyzieu (FR)

(74) Mandataire: Cazes, Jean- Marie, RHONE- POULENC INTERSERVICES Service Brevets Chimie 25, quai Paul Doumer, F-92408 Courbevoie Cédex (FR)

(56) Documents cité: (suite)
D.C., US; A.E. FEIRING: "Chemistry in hydrogen fluoride. 7. A novel synthesis of aryl trifluoromethyl ethers"

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 140 783 B1

LIBER, STOCKHOLM 1986

**Description**

La présente invention concerne un procédé de préparation d'anilines méta-substituées. Plus particulièrement, elle concerne la préparation d'anilines comportant en position méta un groupement trifluorométhoxy ou chlorodifluorométhoxy.

La synthèse d'anilines portant de tels groupements a déjà fait l'objet de plusieurs publications. On peut dire, d'une façon générale, que l'art antérieur concernant ce sujet est constitué de deux groupes distincts de procédés.

Dans un premier groupe, se situent les procédés dont les matières premières sont des dérivés méta-substitués du benzène, lesquels, par transformations successives en conservant la disposition méta, aboutissent à la métatrifluorométhoxyaniline et à la méta(chlorodifluoro)méthoxyaniline correspondantes.

C'est ainsi que dans le Journal of General Chemistry of USSR, 31, 845-52 (19561), YAGUPOLSKII et ses collaborateurs décrivent un procédé de préparation de la métatrifluorométhoxyaniline à partir du chlorure de m-méthoxybenzoyle. Ce procédé présente des inconvénients qui tiennent d'une part à la matière première qui n'est pas facilement disponible au plan industriel et d'autre part au nombre élevé d'étapes (six), ce qui pose des problèmes de rendement. De plus, la dernière étape d'un tel procédé est une dégradation d'Hoffmann dont l'homme de l'art sait bien qu'elle pose de sérieux problèmes au plan de la sécurité. Cet ensemble d'inconvénients rend ce procédé non exploitable au plan industriel.

Une voie identique est décrite par E.L. STROGRYN dans le J.Med. Chem., 16, (12), 1399-1401 pour l'obtention de dérivés chlorodifluorométhoxylés. Les mêmes inconvénients sont à souligner.

Un procédé pour préparer la métatrifluorométhoxyaniline est également décrit par A.E. FEIRING dans le Journal of Organic Chemistry, 44, (1974), exemple 9 du tableau de la page 2908, avec un rendement faible (26 %), par trifluorométhylationde l'amino-3-phénol. Or, il est connu de l'homme du rétier que ce produit de départ n'existe pas en tant que produit commercial et qu'il est même difficile de l'obtenir au laboratoire. Il doit être préparé par diazotation de la métanitroaniline, suivie par l'hydrolyse du sel de diazonium obtenu, le métanitrophénol produit étant ensuite transformé par réduction catalytique en métaaminophénol. Etant donné que les rendements moyens des réactions de diazotation sont d'environ 50 %, le rendement total en métatrifluorométhoxyaniline d'un tel procédé sera très faible (13 % environ).

Le deuxième groupe comprend les procédés dont les produits de départ sont des dérivés para-substitués du benzène. Dans ce cas, un troisième groupement doit être introduit en position méta (par rapport au groupement fluoré) et, donc, le groupe en para devra être éliminé.

C'est ainsi que YAGUPOLSKII et ses collaborateurs, dans le Journal of General Chemistry USSR, 31, 845-52 (1961), décrivent un procédé pour préparer la métatrifluorométhoxyaniline à partir du p-acétylaminophényltrifluorométhyléther (lequel est obtenu par acétylation de la p-trifluorométhoxyaniline). Or, la p-trifluorométhoxyaniline elle-même, n'est pas un produit industriel. Il faut donc la préparer et de ce fait on obtient un procédé faisant intervenir au moins six étapes pour arriver à la métatrifluorométhoxyaniline.

En résumé, compte tenu de l'état de la technique ci-dessus analysé, les synthèses de la métatrifluorométhoxyaniline et de la méta(chlorodifluoro)méthoxyaniline posent de graves problèmes industriels tels que dangers d'explosion (surtout pour les dégradations d'Hoffmann), matières premières non disponibles facilement, grand nombre d'étapes et rendements faibles. De plus un certain nombre de réactifs utilisés dans ces synthèses de l'art antérieur posent des problèmes de toxicité.

Ce qui précède montre bien l'intérêt qu'il y aurait à pouvoir préparer les anilines métasubstituées précitées en partant de matières premières facilement disponibles du point de vue industriel, avec un nombre réduit d'étapes, lesquelles ne doivent pas comporter de risques sur le plan de la sécurité, et permettant d'obtenir de bons rendements.

La demanderesse a découvert un procédé permettant d'atteindre ces objectifs.

L'invention a donc pour objet un procédé de préparation d'anilines comportant en méta un groupement trifluorométhoxy ou chlorodifluorométhoxy, caractérisé en ce que:

- dans une première étape, on fait réagir un orthochlorophénol de formule:

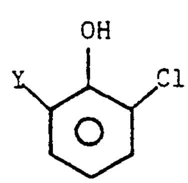

dans laquelle Y représente un atome d'hydrogène ou de chlore, avec un agent de méthylation pour obtenir l'ortho-chloroanisole correspondant;

- dans une deuxième étape, l'orthochloroanisole est soumis à une réaction de chloration pour obtenir le dérivé chloré de formule:

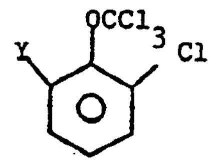

- dans une troisième étape, on soumet le composé obtenu dans la deuxième étape à une réaction de fluoration, de façon à obtenir le dérivé fluoré de formule:

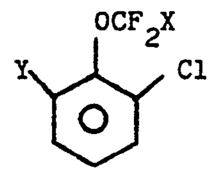

où X représente soit l'atome de chlore, soit l'atome de fluor;

- qui, dans une quatrième étape, par réaction avec un amidure alcalin dans l'ammoniac liquide comme solvant est transformé en aniline de formule:

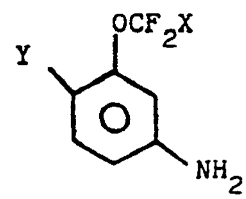

La réaction de méthylation qui constitue la première étape de ce procédé peut être effectuée selon un premier mode de réalisation préférentiel à l'aide de sulfate de diméthyle en présence d'une base.

Selon un deuxième mode de réalisation préférentiel, on peut utiliser comme agent de méthylation le chlorure de méthyle en présence d'une base.

La base de préférence utilisée peut être représentée par la formule BZ où B est un cation d'un métal alcalin ou l'ammonium, et Z est le groupement hydroxyle ou carbonate. La base encore plus préférentiellement mise en oeuvre est la soude.

Selon un premier mode de réalisation préférentiel de la deuxième étape du procédé selon l'invention, la chloration est effectuée à l'aide de chlore gazeux, sous irradiation et en milieu organique, porté au reflux.

On opère de préférence avec une lampe émettant une longueur d'onde comprise entre 250 nm et 500 nm dans le tétrachlorure de carbone.

Un autre mode de réalisation de cette deuxième étape consiste à faire la chloration à l'aide de chlore gazeux, sans solvant, en utilisant le pentachlorure de phosphore comme catalyseur à une température comprise entre 150°C et 250°C.

Pour la troisième étape du procédé selon l'invention deux modes de réalisation sont aussi envisageables. Selon un premier mode, on utilise du trifluorure d'antimoine seul quand X = Cl et du trifluorure d'antimoine ainsi que du pentachlorure d'antimoine comme catalyseur, quand X = F. Cette fluoration peut être effectuée dans les deux cas sans solvant ou avec un solvant comme le dioxanne.

Dans un deuxième mode de réalisation, la fluoration est faite à l'aide d'acide fluorhydrique anhydre.

La quatrième étape est effectuée de préférence sous pression atmosphérique et à la température de reflux de l'ammoniac liquide avec de l'amidure de sodium. Cette étape se caractérise par une sélectivité isomérique de 100 % en méta.

Lors de la méthylation de la première étape on utilise de préférence un excès molaire d'agent de méthylation et de base d'au moins 100 % par rapport à l'orthochlorophénol. On mélange les réactifs à une température comprise entre 0°C et 25°C puis on porte le mélange réactionnel à une température voisine de 100°C.

Dans la deuxième étape, lors de la chloration à l'aide de chlore gazeux en présence de pentachlorure de phosphore on utilise ce dernier en quantité comprise entre 1 et 10 % par rapport à l'orthochloroanisole.

Pour la troisième étape, on utilise toujours un excès molaire de $SbF_3$ par rapport à la matière première, compris de préférence entre 10 et 100 %. L'obtention du composé où X = Cl est réalisée à une température comprise entre 50°C et 150°C. Pour obtenir le composé où X = F, il faut utiliser environ 10 % de $SbCl_5$, par

rapport au trifluorure d'antimoine et chauffer à une température comprise entre 100° C et 150° C.

La fluoration à l'aide d'acide fluorhydrique est effectuée, suivant la nature de X désirée, à une température comprise entre la température ambiante et 200° C, dans un autoclave et sous pression supérieure à la tension de vapeur de l'acide fluorhydrique à la température considérée.

Lorsque l'on désire que X ne soit pas échangé et que l'on veut obtenir une metachlorodifluoromethoxyaniline, la fluoration sera réalisée par exemple entre la température ambiante et 50°C. Lorsque l'on désire que X soit échangé et que l'on veut obtenir une metatrifluorométhoxyaniline, la fluoration sera réalisée par exemple entre 50°C et 200°C. Il est certain que ces limites ne peuvent être déterminées avec précision. Il existe en effet, une zone de température intermédiaire où l'on obtiendra un mélange de dérivés di et trifluorés.

Dans la quatrième étape on utilise de préférence une quantité d'amidure alcalin comprise entre 110 % et 400 % en mole par rapport au composé fluoré.

Les anilines obtenues selon le procédé de l'invention sont utiles comme intermédiaires de synthèse pour la préparation de composés ayant une activité phytosanitaire ou pharmaceutique.

D'autres caractéristiques et avantages de la présente invention apparaitront à la lecture des exemples qui vont suivre.

**Example 1**

Préparation de la méta(trifluorométhoxy)aniline a.) Méthylation de l'orthochlorophénol:

dans un mélange comprenant 2 moles d'orthochlorophénol, 4 moles de soude et 1 litre d'eau, maintenu entre 5° C et 10° C, on ajoute, en 30 minutes, 4 moles de sulfate de diméthyle.

Le mélange réactionnel est ensuite porté à 90° C pendant 30 minutes, puis refroidi à la température ambiante. La phase organique qui décante est d'abord lavée avec une solution diluée de soude, puis à l'eau jusqu'è neutralité.

L'analyse par chromatographie en phase vapeur de cette phase organique séchée et filtrée indique une pureté en o-chloroanisole de 99,6 % et un rendement en o-chloroanisole de 84,6 %.

b) Chloration de l'orthochloroanisole

- chloration photoinduite: dans une solution de 1 mole d'orthochloroanisole dans un litre de tétrachlorure de carbone, portée au reflux et irradiée par une lampe à incandescence de 60 watts, on introduit, en 12 heures, 8,5 moles de chlore. L'avancement de la réaction est suivi par chromatographie en phase vapeur.

Après dégazage du milieu réactionnel à l'azote sec, on distille le tétrachlorure de carbone sous pression atmosphérique, puis le tétrachloro-α, α, α, 2-anisole sous 4 mmHg. Le rendement en tétrachloro-α, α, α, 2-anisole est de 88 %.

- Chloration thermique: dans un mélange agité de 248 g (1,74 mole) de chloro-2 anisole et de 26,1 g de pentachlorure de phosphore (0,125 mole, soit 7 % molaire par rapport au chloroanisole), porté à 195°C, on introduit du chlore gazeux avec un débit de 120 g/h pendant 4 h 50 minutes.

Après distillation, le rendement en chloro-2(trichlorométhoxy)-benzène est de 71 %.

c) Fluoration du chloro-2(trichlorométhoxy)benzène

- A l'aide de trifluorure d'antimoine avec du pentachlorure d'antimoine comme catalyseur: dans un mélange agité de 0,6 mole de trifluorure d'antimoine et 0,035 mole de pentachlorure d'antimoine, maintenu à 40°C, on ajoute goutte à goutte 0,5 mole de tétrachloro-α, α, α, 2-anisole. La température monte è 108° C. On la maintient à cette valeur pendant 20 minutes, puis on distille sous vide et on obtient 45,7 g de chloro-2(trifluorométhoxy)-benzène. Le rendement est de 46,5 %.

A l'aide d'acide fluorhydrique anhydre: dans un autoclave on charge 100 g (100 ml) d'acide fluorhydrique anhydre, puis 20 g (0,08 mole) de chloro-2(trichlorométhoxy)benzène. Après fermeture, on porte sous agitation le mélange réactionnel à 80° C pendant une heure, puis à 140° C pendant 3 h 30 minutes. La pression autogène maximale qui s'établit est de 26 bars. Après réaction, le mélange est ramené à température ambiante puis à pression atmosphérique. Il est versé dans un mélange de glace et d'eau et extrait au chlorure de méthylène.

Après distillation du solvant on obtient 10,53 g (0,054 mole) de chloro-2(trifluorométhoxy)benzène et 2,56 g (0,012 mole) de chloro-2 (chlorodifluorométhoxy)benzène.

Le rendement en chloro-2(trifluorométhoxy)benzène s'établit donc à 67 % et celui en chloro-2(chlorodifluorométhoxy)benzène recyclable, à 15 %.

d) Préparation dela méta(trifluorométhoxy)aniline

Dans un réacteur de 150 ml équipé d'un condenseur et d'un agitateur magnétique, on introduit 50 ml d'ammoniac liquide et 2,9 g (0,075 mole) d'amidure de sodium en suspension dans 2,9 g de toluène.

On introduit, goutte à goutte, 9,8 g de chloro-2-(trifluorome-thoxy)benzène (0,05 mole) au reflux de l'ammoniac liquide.

On maintient l'agitation 20 mn supplémentaires à cette température, puis on rajoute lentement 5,4 g de chlorure d'ammonium pour neutraliser l'amidure de sodium non transformé.

L'ammoniac est évaporé et le mélange est repris par 100 ml de chlorure de méthylène.

Après élimination des sels par lavage, on récupère 1,6 g de chloro-2-(trifluorométhoxy)benzène non transformé et 6,7 g de méta(trifluorométhoxy)aniline.

Le taux de transformation du chloro-2(trifluorométhoxy)benzène est donc de 84 % et la sélectivité en méta(trifluorométhoxy)aniline formée par rapport au chloro-2(trifluorométhoxy)benzène transformé est de 90 %.

**Exemple 2**

**Préparation de la méta(chlorodifluorométhoxv)aniline**

ab)-Les étapes de méthylation du chloro-2 phénol et de chloration du chloro-2 anisole sont identiques à celles de l'exemple 1.

c) Fluoration du chloro-2(trichlorométhoxy)benzène par l'acide fluorhydrique anhydre: cette réaction est conduite avec les mêmes quantités que dans l'exemple 1, mais le mélange réactionnel n'est maintenu que pendant deux heures entre 20 et 30° C sous agitation.

Après un traitement identique à celui décrit dans l'exemple 1, on obtient le chloro-2(chlorodifluorométhoxy)benzène avec un rendement de 87 %.

d) Préparation de la méta(chlorodifluorométhoxy)aniline: un essai, mené dans des conditions identiques à celles de l'exemple 1, à partir de 0,08 mole d'amidure de sodium et 0,04 mole de chloro-2-(chlorodifluorométhoxy)benzène conduit à un taux de transformation du chloro-2(chlorodifluorométhoxy)benzène de 47,5 % et à une sélectivité en méta(chlorodifluorométhoxy)aniline de 19 % par rapport au chloro-2(chlorodifluorométhoxy)benzène transformé.

**Exemple 3**

**Préparation de la (trifluorométhoxy)-3 chloro-4 aniline**

a) Méthylation du dichloro-2,6 phénol: cette étape est effectuée de manière identique à celle de la méthylation du chloro-2-phénol. Le rendement en dichloro-2,6 anisole est de 77 %.

b) Chloration thermique du dichloro-2,6 anisole: dans un mélange agité de 260,2 g (1,47 moles) de dichloro-2,6 anisole et de 15,4 g (0 074 mole, soit 5 % molaire par rapport au dichloro-2,6 anisole) de pentachlorure de phosphore, porté à 195° C, on introduit du chlore gazeux à saturation du milieu (soit environ 50 g/h) pendant 11 h 30 minutes. Après distillation, le dichloro-2,6 (trichlorométhoxy)benzène est obtenu avec un rendement de 28 %.

c) Fluoration du dichloro-2,6(trichlorotéthoxy)benzène à l'aide d'acide fluorhydrique anhydre: dans un autoclave on chargé 100g (100 ml) d'acide fluorhydrique anhydre puis 40 g (0,14 mole) de dichloro-2,6(trichlorométhoxy)benzène. Après fermeture de l'autoclave, on porte, sous agitation, le mélange réactionnel à 140° C, puis progressivement jusqu'à 160° C, pendant six heures. La pression autogène maximale qui s'établit est de 40 bars. Le traitement, aprés réaction, du mélange obtenu est identique à celui utilisé dans l'exemple 1. Le rendement en dichloro-2,6(tri-fluorométhoxy)benzène isolé est de 86 %.

d) Préparation de la (trifluorométhoxy)-3 chloro-4 aniline: un essai, mené dans des conditions identiques à celles de l'exemple 1, à partir de 0,072 mole d'amidure de sodium et 0,03 mole de dichloro-2,6(trifluorométhoxy)benzène conduit à un taux de transformation du dichloro-2,6 (trifluoromèthoxy)benzène de 89 % et à une sélectivité en chloro-4(trifluorométhoxy)-3 aniline de 76 % par rapport au dichloro-2,6 (trifluorométhoxy)benzène transformé.

**Revendications**

1) Procédé de préparation d'anilines comportant en méta un groupement trifluorométhoxy ou chlorodifluorométhoxy, caractèrisé en ce que:

- dans une première étape on fait réagir l'orthochlorophènol de formule

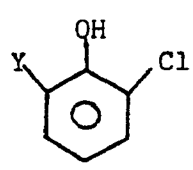

dans laquelle Y représente un atome d'hydrogène ou de chlore, avec un agent de méthylation pour obtenir l'orthochloroanisole correspondant;

- dans une deuxième étape, l'orthochloroanisole est soumis à une réaction de chloration pour obtenir le dérivé chloré de formule:

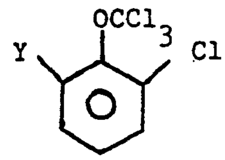

- dans une troisième étape, on soumet le composé obtenu dans la deuxième étape à une réaction de fluoration, de façon à obtenir le dérivé fluoré de formule:

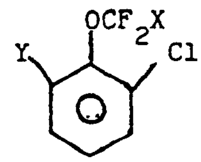

où X représente soit l'atome de chlore, soit l'atome de fluor;

- qui, dans une quatrième étape, par réaction avec un amidure alcalin dans l'ammoniac liquide est transformé en aniline de formule:

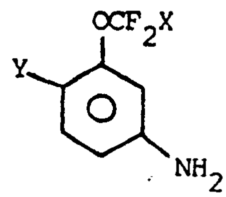

2) Procédé selon la revendication 1, caractérisé en ce que, dans la première étape on utilise comme agent de méthylation le sulfate de diméthyle en présence d'une base.

3) Procédé selon la revendication 1, caractérisé en ce que, dans la première étape on utilise comme agent de méthylation le chlorure de méthyle en présence d'une base.

4) Procédé selon l'une quelconque des revendications 2 et 3, caractérisé en ce que la base est la soude.

5) Procédé selon l'une quelconque des revendications précédentes, caractérisé en ce que dans la deuxième étape la réaction de chloration est effectuée à l'aide de chlore gazeux sous irradiation en milieu solvant organique.

6) Procédé selon l'une quelconque des revendications 1 à 4, caractérisé en ce que dans la deuxième étape la réaction de chloration est effectuée à l'aide de chlore gazeux en présence de pentachlorure de phosphore à une température comprise entre 150 et 250° C.

7) Procédé selon l'une quelconque des revendications précédentes, caractérisé en ce que dans la troisième étape la fluoration est effectuée avec du trifluorure d'antimoine et, quand X = F, en présence de pentachlorure d'antimoine comme catalyseur.

8) Procédé selon l'une quelconque des revendications 1 à 6, caractérisé en ce que dans la troisième étape la fluoration est réalisée avec de l'acide fluorhydrique anhydre.

9) Procédé selon l'une quelconque des revendications précédentes, caractérisé en ce que dans la quatrième étape l'amidure alcalin utilisé est l'amidure de sodium.

**Patentansprüche**

1. Verfahren zur Herstellung von Anilinen mit einer Trifluormethoxy- oder Chlordifluormethoxygruppe in Meta-Stellung, dadurch gekennzeichnet, daß - man in einer ersten Stufe Orthochlorphenol der Formel

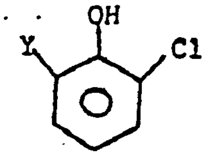

in der Y ein Wasserstoff- oder Chloratom bedeutet, mit einem Methylierungsmittel zu dem entsprechenden Orthochloranisol umsetzt,

- in einer zweiten Stufe das Orthochloranisol einer Chlorierung zu dem Chlorderivat der Formel unterwirft

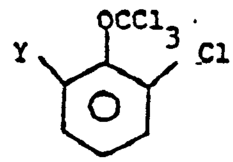

- in einer dritten Stufe die in der zweiten Stufe erhaltene Verbindung einer Fluorierung unterwirft in der Weise, daß man das Fluorderivat der Formel erhält:

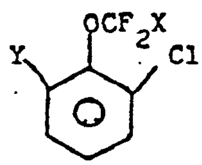

in der X entweder ein Chloratom oder ein Fluoratom bedeutet, welches

- die in einer vierten Stufe durch Umsetzung mit einem Alkaliamid in flüssigem Ammoniak in ein Anilin der Formel

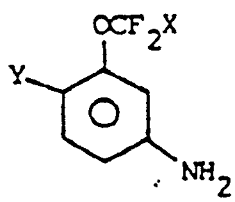

umgewandelt wird.

2. Verfahren nach Anspruch 1, dadurch gekennzeichnet, daß man in der ersten Stufe als Methylierungsmittel Dimethylsulfat in Gegenwart einer Base einsetzt.

3. Verfahren nach Anspruch 1, dadurch gekennzeichnet, daß man in der ersten Stufe als Methylierungsmittel Methylchlorid in Gegenwart einer Base einsetzt.

4. Verfahren nach Anspruch 2 oder 3, dadurch gekennzeichnet, daß die Base Soda ist.

5. Verfahren nach einem der vorangehenden Ansprüche, dadurch gekennzeichnet, daß man in der zweiten Stufe die Chlorierungsreaktion mit Hilfe von gasförmigem Chlor unter Bestrahlung in einem organischen Lösungsmittel durchführt.

6. Verfahren nach einem der Ansprüche 1 bis 4, dadurch gekennzeichnet, daß in der zweiten Stufe die Chlorierungsreaktion mit Hilfe von gasförmigem Chlor in Gegenwart von Phosphorpentachlorid bei einer Temperatur zwischen 150 und 250°C durchgeführt wird.

7. Verfahren nach einem der vorangehenden Ansprüche, dadurch gekennzeichnet, daß man in der dritten Stufe die Fluorierung mit Antimontrifluorid und, wenn X = F, in Gegenwart von Antimonpentachlorid als Katalysator durchführt.

8. Verfahren nach einem der Ansprüche 1 bis 6, dadurch gekennzeichnet, daß man in der dritten Stufe die Fluorierung mit wasserfreier Fluorwasserstoffsäure durchführt.

9. Verfahren nach einem der vorangehenden Ansprüche, dadurch gekennzeichnet, daß man in der vierten Stufe als Alkaliamid Natriumamid einsetzt.

**Claims**

1. Process for the preparation of anilines containing a trifluoromethoxy or chlorodifluoromethoxy group in the meta position, characterized in that:
- in a first stage the ortho-chlorophenol of formula:

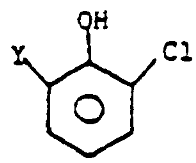

in which Y denotes a hydrogen or chlorine atom, is reacted with a methylating agent to obtain the corresponding ortho-chloroanisole;
- in a second stage, the ortho-chloroanisole is subjected to a chlorination reaction to obtain the chlorine derivative of formula:

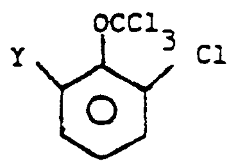

- in a third stage, the compound obtained in the second stage is subjected to a fluorination reaction, so as to obtain the fluorine derivative of formula:

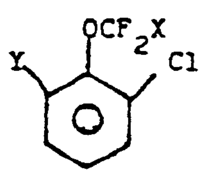

where X denotes either the chlorine atom or the fluorine atom;
- which, in a fourth stage, is converted by reaction with an alkali metal amide in liquid ammonia into an aniline of formula:

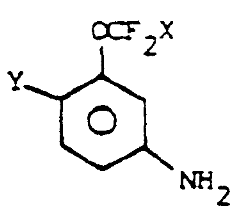

2. Process according to Claim 1, characterized in that dimethyl sulphate is used in the presence of a base as a methylating agent in the first stage:

3. Process according to Claim 1, characterized in that methyl chloride is used in the presence of a base, as a methylating agent in the first stage.

4. Process according to either of Claims 2 and 3 characterized in that the base is sodium hydroxide.

5. Process according to any one of the preceding claims, characterized in that the chlorination reaction in the second stage is carried out using gaseous chlorine under irradiation in an organic solvent medium.

6. Process according to any one of Claims 1 to 4, characterized in that the chlorination reaction in the second stage is carried out using gaseous chlorine in the presence of phosphorus pentachloride at a temperature of between 150 and 250° C:

7. Process according to any one of the preceding claims, characterized in that the fluorination in the third stage is carried out using antimony trifluoride and, when X = F, in the presence of antimony pentachloride as a catalyst.

8. Process according to any one of Claims 1 to 6, characterized in that the fluorination in the third stage is carried out using anhydrous hydrofluoric acid.

9. Process according to any one of the preceding claims, characterized in that the alkali metal amide used in the fourth stage is sodium amide.